(19) Europäisches Patentamt
European Patent Office
Office européen des brevets

(11) Veröffentlichungsnummer: **0 209 783**
**B1**

(12) **EUROPÄISCHE PATENTSCHRIFT**

(45) Veröffentlichungstag der Patentschrift: **20.06.90**

(51) Int. Cl.$^5$: **A 61 K 7/48,** A 61 K 7/06

(21) Anmeldenummer: **86109247.6**

(22) Anmeldetag: **07.07.86**

(54) **Verwendung von Sophoroselipid-lacton zur Bekämpfung von Kopfschuppen und Körpergeruch.**

(30) Priorität: **24.07.85 DE 3526417**

(43) Veröffentlichungstag der Anmeldung:
**28.01.87 Patentblatt 87/05**

(45) Bekanntmachung des Hinweises auf die Patenterteilung:
**20.06.90 Patentblatt 90/25**

(84) Benannte Vertragsstaaten:
**AT BE CH DE FR GB IT LI LU NL SE**

(56) Entgegenhaltungen:
**DE-A-2 938 383**
**GB-A-2 002 369**
**US-A-3 312 684**

**CHEMICAL ABSTRACTS, Band 70, 1969, Seite 319, Zusammenfassung Nr. 11957p, Columbus, Ohio, US; A.P. TULLOCH et al.: "Fermentation of long-chain compounds by Torulopsis apicola. V. Structure and reactions of lactonic and acidic sophorosides of 17-hydroxyoctadecanoic acid", & CAN. J. CHEM. 1968, 46(21), 3337-51**

(73) Patentinhaber: **Wella Aktiengesellschaft**
**Berliner Allee 65**
**D-6100 Darmstadt (DE)**

(72) Erfinder: **Mager, Herbert, Dr.**
**Beaumont 5**
**CH-1700 Fribourg (CH)**
Erfinder: **Röthlisberger, Rudi, Dr.**
**Chemin des Cossettes 2**
**CH-1723 Marly (CH)**
Erfinder: **Wagner, Fritz, Prof. Dr.**
**Hohe Wiese 2**
**D-3300 Braunschweig (DE)**

(56) Entgegenhaltungen:
**CHEMICAL ABSTRACTS, Band 94, 1981, Seite 196, Zusammenfassung Nr. 1950n, Columbus, Ohio, US; S. ITO et al.: "Growth of yeasts on n-alkanes: inhibition by a lactonic sophorolipid produced by Torulopsis bombicola", & AGRIC. BIOL. CHEM. 1980, 44(9), 2221-3**

**EP 0 209 783 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die Erfindung betrifft die Verwendung von Sophoroselipid-lacton zur Bekämpfung von Kopfschuppen oder/und als bakteriostatischer Wirkstoff in Desodorantien.

Es sind bereits zahlreiche Substanzen als Wirkstoffe zur Bekämpfung der Kopfschuppen empfohlen worden. Hierzu gehören durchblutungssteigernde Verbindungen wie Nicotinsäureester, weiterhin Panthenol, kolloidaler Schwefel, Hydroxychinoline, Phenole, quartäre Ammoniumverbindungen, Selensulfid und Pyridinthione.

Von den genannten Verbindungen weisen das 1-Hydroxy-2-pyridinthion und dessen Salze, insbesondere das Zinksalz, eine besonders gute Wirksamkeit gegen Kopfschuppen auf. Aufgrund der in üblichen kosmetischen Lösungsmitteln, wie Wasser und Alkoholen, begrenzten Löslichkeit des Zink-Pyridinthions, kann es jedoch nur unter Schwierigkeiten in klare kosmetische Mittel eingearbeitet werden.

Die bisher als Wirkstoffe zur Behandlung der Kopfschuppen vorgeschlagenen Verbindungen können hinsichtlich ihrer Wirksamkeit gegen Kopfschuppen, in toxikologischer und dermatologischer Hinsicht oder — wie im Falle des Zink-Pyridinthions — wegen der Scherlöslichkeit in kosmetischen Lösungsmitteln den gestellten Anforderungen nicht völlig zufriedenstellend genügen.

Mikroorganismen sind für die Entstehung unangenehmer Körpergerüche verantwortlich. Desodorierend wirkende kosmetische Mittel enthalten deshalb Substanzen, welche das Wachstum der Bakterien hemmen oder verhindern. Als Beispiele seien Hexachlorophen, Dichlor-m-xylenol und Chlorhexidin genannt. Weitere bakterizide oder bakteriostatische Verbindungen sind in J. S. Jellinek, Kosmetologie, Dr. A. Hüthig Verlag, 3. Auflage (1975), Seite 119 ff. und 401 ff. erwähnt.

Die Desodoranswirkstoffe sind jedoch ebenfalls in dermatologischer und toxikologischer Hinsicht nicht völlig unbedenklich.

Aufgabe der Erfindung ist es daher, ein kosmetisches Mittel zur Bekämpfung von Kopfschuppen sowie Körpergeruch zur Verfügung zu stellen, das die obenerwähnten Nachteile nicht aufweist.

Hierzu wurde nun gefunden, daß durch die Verwendung mindesten eines Sophoroselipid-lactons der allgemeinen Formel I

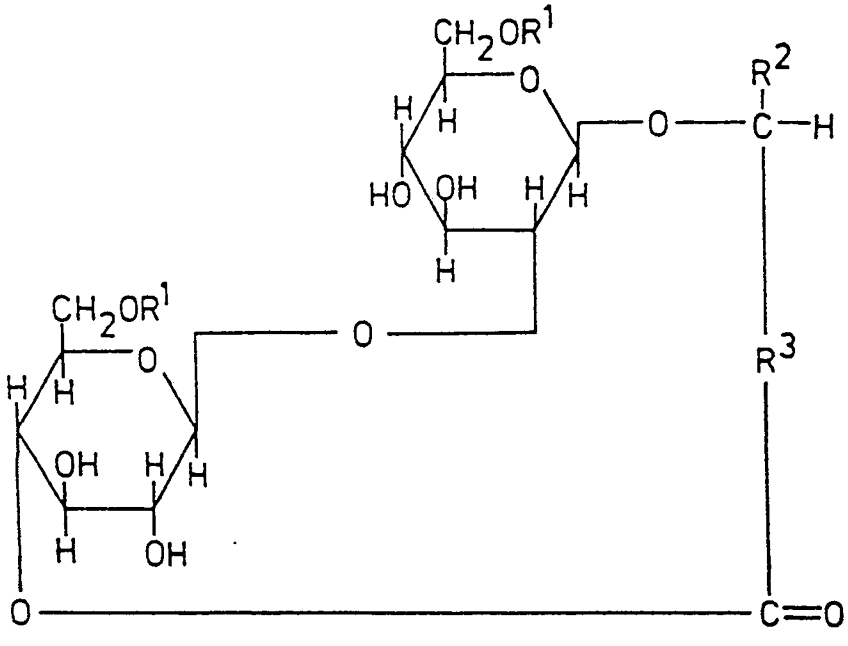

(I),

worin $R^1$ Wasserstoff oder eine Acetylgruppe bedeutet und $R^2$ Wasserstoff oder einem Alkylrest mit 1 bis 9 Kohlenstoffatomen darstellt, wenn $R^3$ ein gesättigter Kohlenwasserstoffrest mit 7 bis 16 Kohlenstoffatomen ist, oder $R^2$ Wasserstoff oder eine Methylgruppe darstellt, wenn $R^3$ ein ungesättigter Kohlenwasserstoffrest mit 13 bis 17 Kohlenstoffatomen ist, in kosmetischen Mitteln zur Bekämpfung von Kopfschuppen und/oder als bakteriostatischer Wirkstoff in Desodorantien, die gestellten Anforderungen in hervorragender Weise erfüllt werden.

Besonders bevorzugt ist die Verwendung eines Gemisches aus Sophoroselipid-lactonen der Formel I, worin bei 90 Gew.% $R^1$ Wasserstoff oder eine Acetylgruppe bedeutet, $R^2$ Wasserstoff oder einem Alkylrest mit 1 bis 9 Kohlenstoffatomen darstellt und $R^3$ ein gesättigter Kohlenwasserstoffrest mit 7 bis 16 Kohlenstoffatomen ist, und bei 10 Gew.% $R^1$ Wasserstoff oder eine Acetylgruppe bedeutet, $R^2$ Wasserstoff oder eine Methylgruppe darstellt und $R^3$ ein ungesättigter Kohlenwasserstoffrest mit 13 bis 17 Kohlenstoffatomen ist.

Das Sophoroselipid-lacton der Formel I ist erhältlich durch Fermentierung von Glucose und Sojaöl mit

Mikroorganismen. Die Bildung von Sophorselipiden durch Mikroorganismen ist literaturbekannt und wurde unter anderem von A. P. Tulloch [1] beschrieben. Als Mikroorganismen werden zum Beispiel die Hefen Torulopsis apicola oder Torulopsis bombicola verwendet. Das entstehende Produkt stellt ein Gemisch aus verschiedenen offenkettigen Sophoroselipiden sowie von Sophoroselipid-lactonen dar, aus dem durch eine geeignete Aufarbeitung (siehe Beispiel 1) die Sophoroselipid-lactone isoliert werden können. Nach Göbbert et al.[2] entstehen bei der Fermetierung von Glucose und Sojaöl mit wachsenden Hefekulturen etwa 90 Gew.% gesättigte und etwa 10 Gew.% ungesättigte Sophoroselipide. Dagegen erzielt man mit ruhenden Hefezellen ein Produkt mit der genau entegegengesetzen prozentualen Zusammensetzung.

In den für kosmetische Mittel in Betracht kommenden Konzentrationen weist das Sophoroselipid-lacton eine ausgezeichnete Wirksamkeit gegen Kopfschuppen auf. Zudem kann es wegen seiner sehr guten Löslichkeit in den für kosmetische Präparate üblichen Lösungsmitteln, wie Wasser und Wasser-Alkohol-Gemischen, in den meisten kosmetischen Zubereitungsformen Verwendung finden. Gegenüber bekannten Mitteln gegen Kopfschuppen, wie zum Beispiel Zink-Pyridinthion, hat das Sophoroselipid-lacton der Formel I wesentlich günstigere toxikologische Eigenschaften. Eine Belastung des Haares tritt ebenfalls nicht auf.

Überraschenderweise wurde zudem gefunden, daß das Sophoroselipid-lacton der Formel I in bezug auf eine Reihe von Mikroorganismen eine bakteriostatische Wirkung aufweist. Im Gegensatz zu bekannten Wirkstoffen in Desodorantien, die bei einem Teil der Anwender Hautirritationen hervorrufen können, ist das Sophoroselipid-lacton physiologisch unbedenklich und ausgezeichnet hautverträglich.

Da das Sophoroselipid-lacton der Formel I zudem sehr gute rückfettende und hautpflegende Eigenschaften hat, beugt es einer Austrocknung der Haut vor und vermittelt ein angenehmes Hautgefühl.

Die das Sophoroselipid-lacton der Formel (I) enthaltenden kosmetischen Mittel können in beliebigen, für die Haar-und Hautbehandlung geeigneten Zubereitungsformen, zum Beispiel als klare oder trübe Lösungen, Dispersionen oder Emulsionen, vorzugsweise aber als klare Zubereitung vorliegen.

Als Beispiele für kosmetische Mittel, die ein Sophoroselipid-lacton der Formel I enthalten, seien Desodorantien, Kopfhaut-Pflegemittel, Anti-Schuppen-Mittel, Rinses, Körperlotionen, Haarkuren, haarwässer, Shampoos und Schaum- oder Duschbäder erwähnt.

Es handelt sich dabei um Zubereitungen, die je nach ihrem Anwendungszweck für kürzere oder längere Zeit auf dem Haar oder der Haut verbleiben. Durch ihren Gehalt an den beschriebenen Verbindungen der Formel I wird hierbei gleichzeitig eine Schuppenbehandlung sowie eine Desodorierung und eine Pflege der Haut und des Haares erreicht. Es ist jedoch auch möglich, Zubereitungen herzustellen, die hauptsächlich oder ausschließlich dem Ziele einer Schuppenbekämpfung oder einer Desodorierung dienen.

Das Sophoroselipid-lacton der Formel (I) wird in den hier beschriebenen kosmetischen Mitteln in einer Konzentration von 0,1 bis 5 Gew.%, vorzugsweise von 0,5 bis 3 Gew.%, verwendet.

Die Zusammensetzung dieser kosmetischen Mittel stellt eine Mischung der Verbindung der Formel I mit den für solche Zubereitungen üblichen, physiologisch verträglichen Bestandteilen, wie Träger- und Zusatzstoffen, dar.

Übliche Träger- und Zusatzstoffe in Lösungen, Emulsionen oder Dispersionen sind zum Beispiel Lösungsmittel wie Wasser, niedere aliphatische Alkohole, beispielsweise Ethanol, Propanol oder Glykole wie Glycerin und Propylenglykol, weiterhin Netzmittel oder Emulgatoren aus den Klassen der anionischen, kationischen, amphoteren oder nicht ionogenen oberflächenaktiven Substanzen, wie Fettalkoholsufate, Alkylsulfonate, Alkylbenzolsulfonate, Alkyltrimethylammoniumsalze, Alkylbetaine, oxethylierte Fettalkohole, propoxylierte Fettalkohole, oxethylierte Nonylphenole, Fettsäurealkanolamide, oxethylierte Fettsäureester, ferner Verdicker wie höhere Fettalkohole, Fettsäureester, Stärke, Cellulosederivate, Vaseline, Stearin, Ceresin, Paraffinöl und Fettsäuren, außerdem Pflegestoffe wie Lanolin, Lanolinderivate, Aminosäuren, Aminosäurederivate, Chloresterin, Pantothensäure, Sorbit, Betain, Mandlöl, Avocadoöl, Weizenkeimöl, Bienenwachs, Walrat und Kamillenextrakt, des weiteren kosmetische Harze wie Polyvinylpyrrolidon, Polyvinylacetat, Polyvinylalkohol oder Polyacrylverbindungen wie Acrylsäurebeziehungsweise Methacrylsäurepolymerisate, basische Polymerisate von Estern aus diesen beiden Säuren und Aminoalkoholen beziehungsweise deren Salze oder Quaternisierungsprodukte, Polyacrylnitril, Polyvinyllactame, Copolymerisate aus derartigen Verbindungen, Derivaten von natürlichen Polymeren wie Chitosan oder Chitosanderivate, sowie Farbstoffe, Parfümöle und Treibgase, ferner anorganische Salze wie Natriumchlorid und Natriumcarbonat sowie Mittel zur Einstellung des pH-Wertes wie z.B. Natriumhdyroxid und Zitronensäure.

Die Herstellung der kosmetischen Zubereitung erfolgt in der für derartige Präparate üblichen Weise, indem die Verbindungen der Formel I mit den als Trägerstoff dienenden Bestandteilen vermischt werden und danach mit den weiteren Bestandteilen der Mittel zum fertigen Endprodukt konfektioniert werden.

Die folgenden Beispiele sollen die Erfindung näher erläutern.

---

[1] A. P. Tulloch, J. F. T. Spencer and P. A. J. Gorin, Can. J. Chem. *40*, 1326 (1962)

[2] U. Göbbert, S. Lang und F. Wagner, Biotechnology Letters *6* (4), 225 (1984)

## HERSTELLUNGSBEISPIEL

### Beispiel 1

Herstellung von Sophoroselipid-lacton der Formel I durch Fermentierung von Sojaöl mit wachsenden Hefezellen.

a) Als Mikroorganismus wird Torulopsis bombicola ATTC 22 214 verwendet, das in einem YM Agar-Kulturmedium in Schrägkultur 24 Stunden lang gezüchtet worden ist.

b) Das Nährmedium beseht aus deionisiertem Wasser, in dem 10 Gew.% Glucose, 1 Gew.% technischer Hefeextrakt und 0,1 Gew.% Harnstoff enthalen sind. Es wird bei 121°C und einem pH-Wert von 6,0 30 Minuten lang sterilisiert.

c) 100 ml des Nährmediums werden in einem 500 ml Erlenmeyerkolben mit Torulopsis bombicola beimpft und anschließend 48 Stunden lang bei 30°C auf einer Rotationsschüttelmaschine bei 100 Umdrehungen pro Minute kultiviert.

d) Von dieser Fermentationslösung werden sodann jeweils 10 ml in 10 Erlenmeyerkolben von jeweils 2000 ml Inhalt gegeben, welche jeweils 500 ml Nährmedium enthalten. Die Kultivierung erfolgt 24 Stunden lang analog Abschnitt c).

e) Man gibt die auf diesem Wege erhaltenen 5000 ml Fermentationslösung in einen Bioreaktor und fügt nach 19, 43 und 67 Stunden Inkubationszeit jeweils 610 g Sojaöl zu.

Reaktorbedingungen

Arbeitsvolumen: 50 l
Rührsystem: Umwurf
Belüftungsrate: 1 VVM bei 2 bar Vordruck
Rührerdrehzahl: 1500 Umdrehungen/Minute
Temperatur: 30°C, nach 43 Stunden 23°C
pH-Wert: 6,0
Reaktionsdauer: 140 Stunden

f) Das Kulturgemisch wird anschließend mit einer Durchlaufzentrifuge bei 17000 Umdrehungen pro Minute zentrifugiert. Das Kulturfiltrat und der Kulturrückstand werden getrennt jeweils mit Essigsäureethylester extrahiert, die organischen Extrakte vereinigt und das Lösungsmittel im Vakuum entfernt. Es verbleiben 2000 g Rückstand, der zu 95 Gew.% aus Sophoroselipiden besteht. Nach der Umkristallisation des Rückstandes aus Ethanol erhält man 700 g eines Gemisches aus Sophoroselipid-lactonen der Formel I, worin bei etwa 90 Gew.% $R^1$ Wasserstoff oder eine Acetylgruppe bedeutet, $R^2$ Wasserstoff oder einen Alkylrest mit 1 bis 9 Kohlenstoffatomen darstellt und $R^3$ ein gesättigter Kohlenwasserstoffrest mit 7 bis 16 Kohlenstoffatomen ist, und bei etwa 10 Gew.% $R^1$ Wasserstoff oder eine Acetylgruppe bedeutet, $R^2$ Wasserstoff oder eine Methylgruppe darstellt und $R^3$ ein ungesättigter Kohlenwassersoffrest mit 13 bis 17 Kohlenstoffatomen ist.

## ANWENDUNGSBEISPIELE

### Beispiel 2

Haarwasser gegen Schuppen

| | | |
|---|---|---|
| 1,5 | g | Sophoroselipid-lacton nach Beispiel 1 |
| 50,0 | g | Isopropanol |
| 0,2 | g | Parfümöl |
| 0,1 | g | Farbstoff |
| 48,2 | g | Wasser |
| 100,0 | g | |

### Beispiel 3

Deodorantstift

| | | |
|---|---|---|
| 2,5 | g | Sophoroselipid-lacton nach Beispiel 1 |
| 1,5 | g | Triisopropanolaminmyristat |
| 80,0 | g | Ethylalkohol, 95%ig |
| 5,0 | g | Stearinsäure |
| 0,8 | g | Natriumhydroxyd |
| 2,0 | g | Diethylenglycolmonoethylether |
| 3,0 | g | Glycerin |
| 0,8 | g | Parfümöl |
| 4,4 | g | Wasser |
| 100,0 | g | |

Man reibt die Haut, zum Beispiel unter den Achseln, mit dem Deodorantstift ein. Störender Geruch wird beseitigt, eine Hautirritation tritt nicht auf.

Beispiel 4

Roll-On-Deodorant

| | | |
|---|---|---|
| 3,0 | g | Sophoroselipid-lacton nach Beispiel 1 |
| 4,0 | g | Hydroxyethylcellulose |
| 3,0 | g | Cetylalkohol, propoxyliert |
| 1,0 | g | Natriumcarbonat |
| 45,0 | g | Ethanol, 95%ig |
| 1,0 | g | Parfümöl |
| 43,0 | g | Wasser |
| 100,0 | g | |

Beispiel 5

Rinse gegen Schuppen

| | | |
|---|---|---|
| 1,5 | g | Sophoroselipid-lacton nach Beispiel 1 |
| 6,0 | g | Cetylstearylalkohol |
| 3,0 | g | Copolyemrisat aus Vinylimidazoliummethochlorid und Vinylpyrrolidon (95:5), 40%ige wäßrige Lösung |
| 1,5 | g | 1,2-Propylenglykol |
| 0,2 | g | Zitronensäure |
| 0,4 | g | Parfümol |
| 87,4 | g | Wasser |
| 100,0 | g | |

Beispiel 6

Shampoo gegen Schuppen

| | | |
|---|---|---|
| 2,0 | g | Sophoroselipid-lacton nach Beispiel 1 |
| 40,0 | g | Laurylalkohol-diglykolethersulfat-Natriumsalz, 28%ige wäßrige Lösung |
| 4,0 | g | Natriumchlorid |
| 0,8 | g | Parfümöl |
| 53,2 | | gWasser |
| 100,0 | g | |

Beispiel 7

Duschbad (desodorierend)

| | | |
|---|---|---|
| 3,0 | g | Sophoroselipid-lacton nach Beispiel 1 |
| 16,0 | g | Laurylalkohol-diglykolethersulfat-Natriumsalz, 28%ige wäßrige Lösung |
| 15,0 | g | Acylglutamat 30%ig |
| 0,8 | g | Parfümöl |
| 65,2 | g | Wasser |
| 100,0 | g | |

Beispiel 8

Schaumbad (desodorierend)

| | | |
|---|---|---|
| 2,5 | g | Sophoroselipid-lacton nach Beispiel 1 |
| 47,0 | g | Gemisch aus Natrium- und Manesiumlaurylethersulfat, 27%ig (Texapon ASV der Firma Henkel, Düsseldorf) |
| 2,0 | g | Polyoxyethylensorbitanmonolaurat |
| 2,0 | g | Kamillenextrakt, wasserlöslich |
| 3,0 | g | Natriumchlorid |
| 1,0 | g | Parfümöl |
| 42,5 | g | Wasser |
| 100,0 | g | |

Alle in der vorliegenden Anmeldung angegebenben Prozentzahlen stellen Gewichtsprozente dar.

**Patentansprüche**

1. Verwendung von Sophoroselipid-lacton der allgemeinen Formel(I)

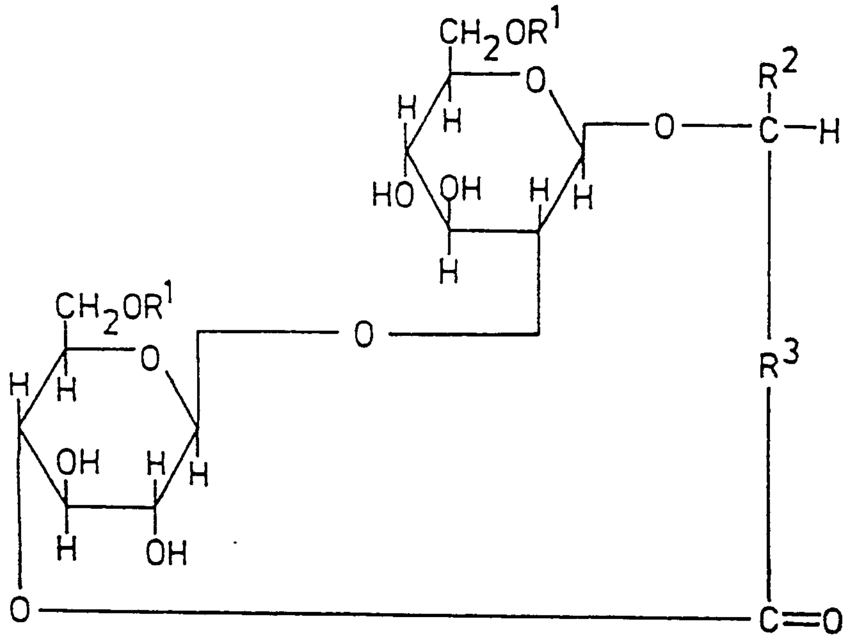

(I),

worin $R^1$ Wasserstoff oder eine Acetylgruppe bedeutet und $R^2$ Wasserstoff oder einen Alkylrest mit 1 bis 9 Kohlenstoffatomen darstellt, wenn $R^3$ ein gesättigter Kohlenwasserstoffrest mit 7 bis 16 Kohlenstoffatomen ist, oder $R^2$ Wasserstoff oder eine Methylgruppe darstellt, wenn $R^3$ ein ungesättigter Kohlenwasserstoffrest mit 13 bis 17 Kohlenstoffatomen ist, in kosmetischen Mitteln zur Bekämpfung von Kopfschuppen.

2. Verwendung von Sophoroselipid-lacton de allgemeinen Formel(I)

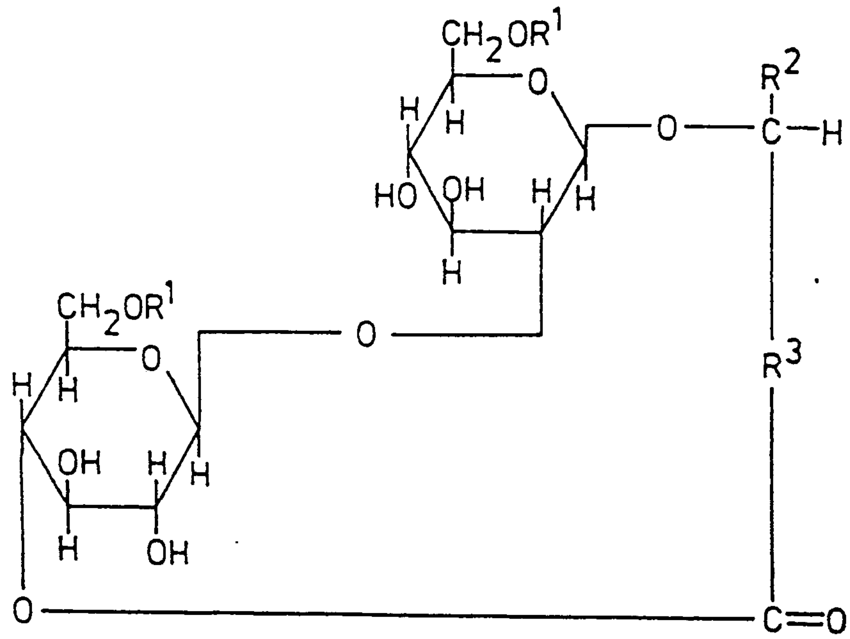

(I),

worin $R^1$ Wasserstoff oder eine Acetylgruppe bedeutet und $R^2$ Wasserstoff oder einen Alkylrest mit 1 bis 9 Kohlenstoffatomen darstellt, wenn $R^3$ ein gesättigter Kohlenwasserstoffrest mit 7 bis 16 Kohlenstoffatomen ist, oder $R^2$ Wasserstoff oder eine Methylgruppe darstellt, wenn $R^3$ ein ungesättigter Kohlenwasserstoffrest mit 13 bis 17 Kohlenstoffatomen ist, als bakteriostatischer Wirkstoff in Desodorantien.

3. Verwendung nach Anspruch 1 oder 2, dadurch gekennzeichnet, daß das Sophoroselipid-lacton der Formel (I) in Form eines Gemisches vorliegt, worin bei 90 Gew.% $R^1$ Wasserstoff oder eine Acetylgruppe bedeutet, $R^2$ Wasserstoff oder einen Alklyrest mit 1 bis 9 Kohlenstoffatomen darstellt und $R^3$ ein gesättiger

Kohlenwasserstoffrest mit 7 bis 16 Kohlenstoffatomen ist, und bei 10 Gew.% $R^1$ Wasserstoff oder eine Methylgruppe darstellt und $R^3$ ein ungesättigter Kohlenwasserstoffrest mit 13 bis 17 Kohlenstoffatomen ist.

4. Verwendung nach einem der Ansprüche 1 bis 3, dadurch gekennzeichnet, daß das Sophoroselipidlacton der Formel (I) in einer Gesamtmenge von 0,1 bis 5 Gew.% eingesetzt wird.

**Revendications**

1. Utilisation de la sophoroselipide-lactone repondant à la formule générale (I)

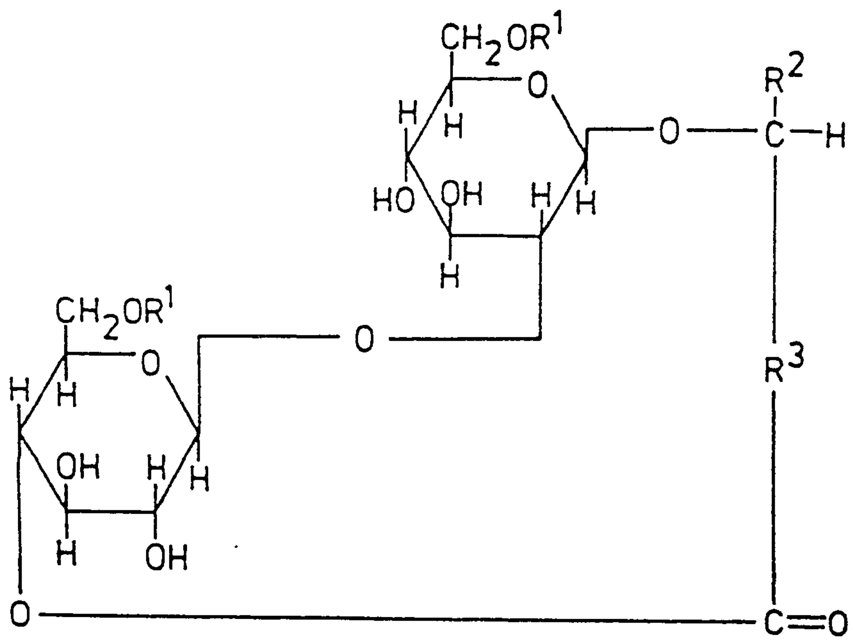

(I),

dans laquelle $R^1$ représente l'hydrogène ou un groupe acétyle et $R^2$ l'hydrogène ou un reste alkyle comportant 1 à 9 atomes de carbone, lorsque $R^3$ est un reste hydrocarboné saturé à 7 à 16 atomes de carbone, ou bien $R^2$ représente l'hydrogène ou un groupe méthyle, lorsque $R^3$ est un reste hydrocarboné insaturé à 13 à 17 atomes de carbone, dans les compositions cosmétiques pour combattre les pellicules.

2. Utilisation de la sophoroselipide-lactone repondant à la formule générale (I)

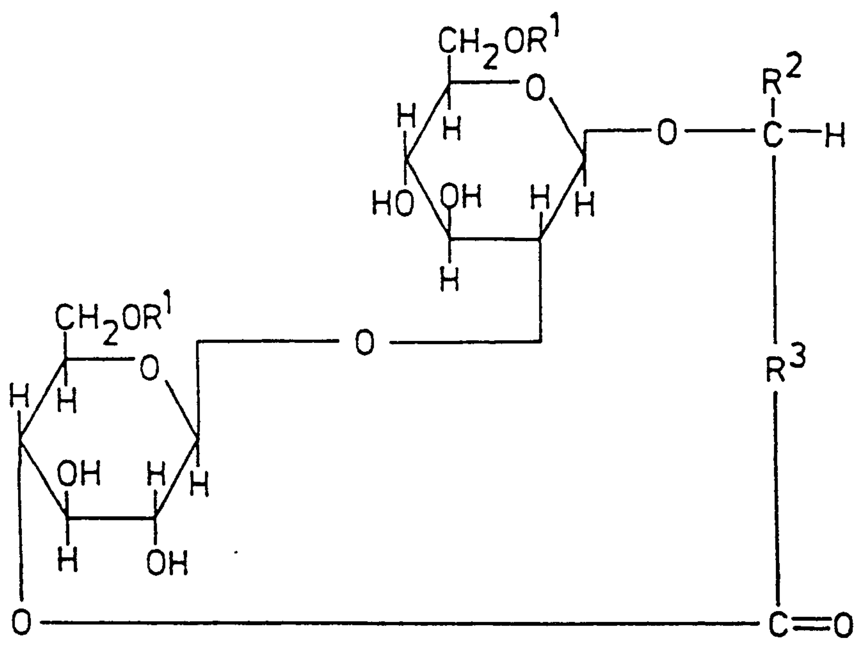

(I),

dans laquelle $R^1$ représente l'hydrogène ou un groupe acétyle et $R^2$ l'hydrogène ou un reste alkyle comportant 1 à 9 atomes de carbone, lorsque $R^3$ est un reste hydrocarboné saturé à 7 à 16 atomes de

carbone, ou bien $R^2$ représente l'hydrogène ou un groupe méthyle, lorsque $R^3$ est un reste hydrocarboné insaturé à 13 à 17 atomes de carbone, comme agent bactériostatique dans des déodorants.

3. Utilisation selon la revendication 1 ou 2, caracterisé en ce que la sophoroselipide-lactone de la formule (I) se présente sous la forme d'un mélange, dans lequel dans 90% en poids $R^1$ représente hydrogène ou un groupe acétyle, $R^2$ est hydrogène ou un groupe alcoyle comportant 1 à 9 atomes de carbone et $R^3$ est un groupe alcoyle saturé comportant 7 à 16 atomes de carbone, et dans 10% eu poids $R^2$ représente hydrogène ou $CH_3$ et $R^3$ est un groupe alcoyle insaturé compostant 13 à 17 atomes de carbone.

4. Utilisation selon l'une des revendications 1 à 3, caracterisé en ce que la sophoroselipide-lactone de la formule (I) est employée dans une quantité totale de 0,1 à 5% en poids.

**Claims**

1. Use of sophorolipid-lactone of the formula (I)

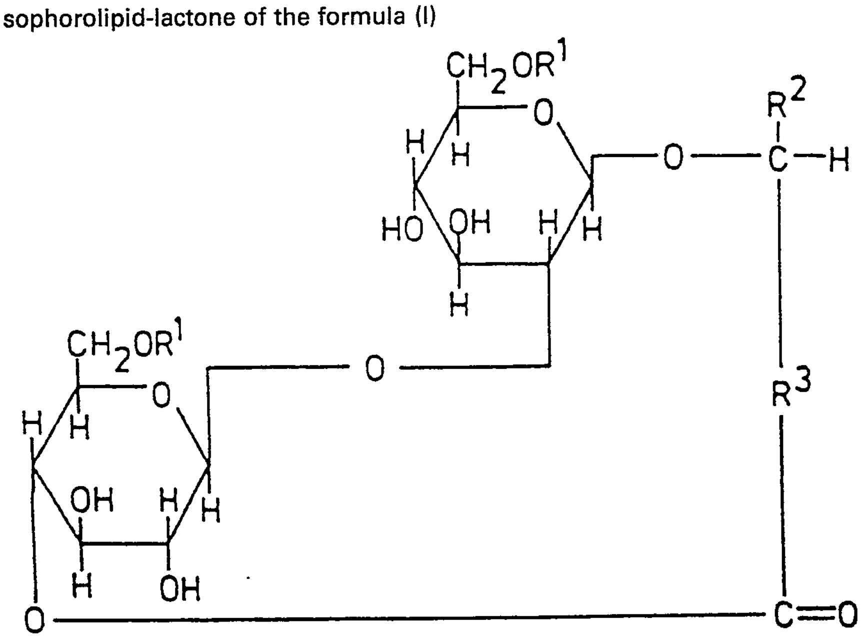

(I),

wherein $R^1$ represents hydrogen or an acetyl group and $R^2$ represents hydrogen or an alkyl group having 1 to 9 carbon atoms, provided that $R^3$ represents a saturated hydrocarbon group having 7 to 16 carbon atoms, or $R^2$ represents hydrogen or a methyl group, provided that $R^3$ represents an unsaturated hydrocarbon group having 13 to 17 carbon atoms, in cosmetic compositions for the treatment of dandruff.

2. Use of sophorolipid-lactone of the formula (I)

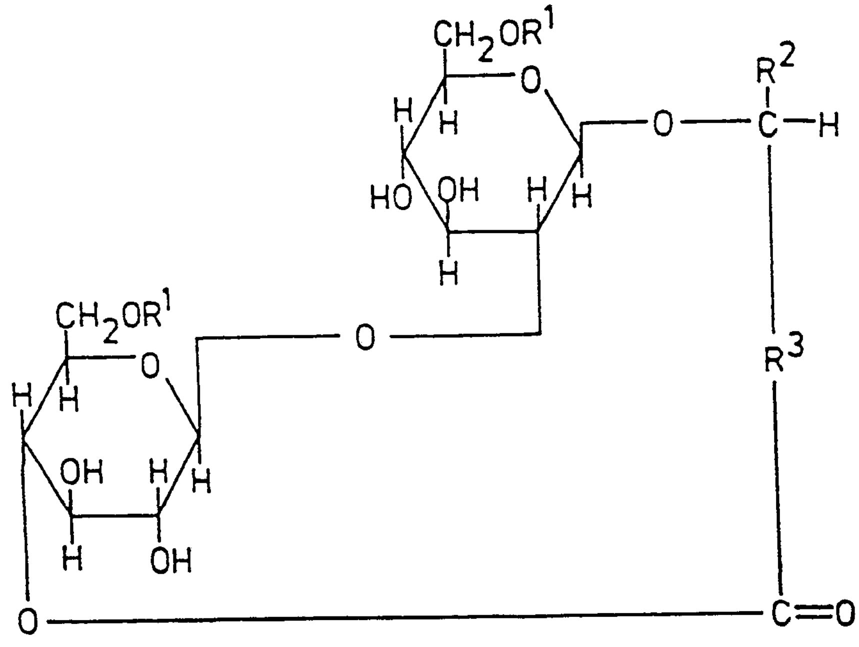

(I),

wherein $R^1$ represents hydrogen or an acetyl group and $R^2$ represents hydrogen or an alkyl group having 1

to 9 carbon atoms, provided that $R^3$ represents a saturated hydrocarbon group having 7 to 16 carbon atoms, or $R^2$ represents hydrogen or a methyl group, provided that $R^3$ represents an unsaturated hydrocarbon group having 13 to 17 carbon atoms, as a bacteriostatic active ingredient in deodorants.

3. Use as claimed in Claim 1 or 2, characterized in that the soporolipidlactone of the formula (I) is in the form of a mixture, wherein in 90% by weight $R^1$ represents hydrogen or an acetyl group, $R^2$ represents hydrogen or an alkyl group and $R^3$ represents a saturated hydrocarbon group having 7 to 16 carbon atoms, and in 10% by weight $R^2$ represents hydrogen or a methyl group and $R^3$ represents an unsaturated hydrocarbon group having 13 to 17 carbon atoms.

4. Use as claimed in any one of Claims 1 to 3, characterized in that the sophorolipid-lactone of the formula (I) is used in a total quantity of 0,1 to 5% by weight.